# EUROPEAN PATENT APPLICATION

(11) **EP 2 213 643 A1**
(43) Date of publication of application: **04.08.2010**
(21) Application number: 09001150.3
(22) Date of filing: 28.01.2009
(51) Int. Cl.: C07C 41/09, C07C 43/215, C07F 17/02

(54) **Process for the preparation of an allyl aryl ether by catalytic o-allylation**

(71) Applicant: Hexion Specialty Chemicals Research Belgium S.A., 1348 Ottignies Louvain-la-Neuve (BE)
(72) Inventor: Postma, Ron, 3196 KK Vondelingenplaat RT (NL)

(57) **Abstract**

The invention provides a process for the preparation of an allyl aryl ether comprising the O-allylation of an aromatic hydroxyl containing compound with an allyl source in the presence of a catalyst, wherein the catalyst is a transition metal complex with a bidentate diphosphine ligand, and wherein the bidentate diphosphine ligand has 2 to 4 bridging atoms between the phosphorus atoms and wherein at least one of the bridging atoms is substituted.

This invention further provides novel transition metal complexes that may be used in the above process. This invention further provides a process for the preparation of epoxy resins wherein as intermediate use is made of the allyl aryl ethers prepared by the process of the invention.

## Description

### Technical Field

The present invention relates to a process for the preparation of an allyl aryl ether by catalytic O-allylation of aromatic hydroxyl containing compounds ("phenols"). This may be achieved by reaction of an allyl source such as allyl alcohol or a similar reactant in the presence of a suitable catalyst. The selective O-allylation over the C-allylation is achieved by the use of metal complexes with certain bidentate diphosphine ligands as catalyst.

### Background Art

Allyl ethers of polyphenols are useful for preparing epoxy compounds low in aliphatic halogen content. From US 4740330 it is known that the aromatic hydroxyl groups of aromatic hydroxyl-containing compounds can be O-allylated to greater than 95% conversion by reacting the alkali metal salt of said aromatic hydroxyl-containing compounds with an allyl halide such as allyl chloride in the presence of a polar aprotic solvent such as dimethylformamide. These compounds are useful in preparing epoxy resins containing a low halogen content. The process of this reference requires the use of an allyl halide and thus generates halide salts as by-products. From an environmental and atom-efficiency point of view, this route is not attractive.

Likewise US 7049388 discloses a process for manufacturing an alpha -dihydroxy derivative from an aryl allyl ether wherein such alpha -dihydroxy derivative can be used to prepare an alpha -halohydrin intermediate and an epoxy resin prepared therefrom including epoxidizing an alpha -halohydrin intermediate produced from a halide substitution of an alpha -dihydroxy derivative which has been obtained by a dihydroxylation reaction of an aryl allyl ether in the presence of an oxidant or in the presence of an oxidant and a catalyst. The starting material in the dihydroxylation process is an aryl allyl ether or mixture of aryl allyl ethers. In Example 1 the conversion of bisphenol A to bisphenol A diallyl ether is described, which was synthesized as described in US4740330 mentioned above. This process therefore suffers from the same disadvantages as that described in US4740330.

From VAN DER DRIFT, Robert C., et al. Two reactions of allylic alcohols catalysed by ruthenium cyclopentadienyl conplexes with didentate phosphine ligands: isomerisation and ether formation. Journal of Molecular Catalysis A: Chemical. 2000, vol.159, p.163-177. it is known that ethers may be formed from heterocoupling of allyl alcohol with aromatic alcohols. In particular [RuClCp(*o-*MeO-dppe)], wherein Cp stands for η⁵-cyclopentadienyl and o-MeO-dppe stands for 1,2-bis(di(*ortho*-methoxyphenyl)phosphino)ethane, has proven to be very active in ether formation. Thus phenol was converted into allyl phenyl ether; *p-*cresol was converted into allyl (*p*-methylphenyl) ether (AMPE); *p*-MeO-phenol was converted into allyl (*p*-methoxyphenyl) ether; 2,4,6-trimethylphenol was converted into allyl (2,4,6-trimethylphenyl) ether and 2-naphthol was converted into allyl 2-naphtyl ether. Turnover frequencies (TOF) of up to 875 h⁻¹ were achieved. On the other hand, under the reaction conditions applied, at 100 degrees Centigrade, AMPE underwent a Claisen rearrangement to form a mixture of several ring allylated cresols and ethers. C-allylation, however, is undesirable.

In SABURI, Hajime, et al. Catalytic Dehydrative Allylation of Alcohols. Angew. Chem., Int. Ed. Engl.. 2005, vol.44, p.1730-1732. the salt waste-free allylation of alcohols is described, using allyl alcohol as allyl source. Water is thus the only coproduct, making this dehydrative allylation both efficient, environmentally friendly and simple to operate. It too employs a cationic ruthenium cyclopentadienyl complex. On the other hand, the direct allylation of phenol with allyl alcohol (2-propen-1-ol), catalyzed by CpRuPF₆]-2-pyridinecarboxylic acid derivative combined systems was low, likely as a result of the low nucleophilicity of the phenol and the reversibility of the catalysis.

Not only Ruthenium has been used for etherification of allyl alcohols with phenols. In SATOH, Tetsuya, et al. Palladium-Catalyzed Etherification of Allyl Alcohols Using Phenols int he Presence of Titanium(IV) Isopropoxide. J. org. chem.. 1997, vol.62, p.4877-4879. the palladium-catalyzed nucleophilic substitution of allyl alcohols in the presence of titanium(IV) isopropoxide is described. The catalyst requires a stoichiometric amount of promoter to achieve O-allylation, generating large amounts of waste.

Likewise, in KIM, Hahn, et al. A Mild and Efficient Method for the Stereoselective Formation of C-O Bonds: Palladium-Catalyzed Allylic Etherification Using Zinc(II) Alkoxides. Organic Letters. 2002, vol.4, no.24, p.4369-4371. a highly chemo- and stereoselective palladium-catalyzed allylic etherification reaction is described. In this reference aliphatic alcohols are reacted with allylic acetates. Again, the catalyst requires stoichiometric amounts of zinc alkoxide as promoter, generating large amounts of waste. No information can be found on the O-allylation of aromatic alcohols.

In BRUNEAU, Christian, et al. Pentamethylcyclopentadienyl-Ruthenium Catalysts for Regio- and enantioselective allylation of Nucleophiles. Chem. Eur. J.. 2006, vol.12, p.5178-5187. the allylation of phenols with e.g., cinnamyl chloride or with allyl benzyl chloride was investigated. Various catalysts were tested. The yield of O-allylated product, however, is low; apparently the C-C allylation is highly favoured.

C-allylation is known from a series of references. For instance palladium-catalyzed C-allylation is known from KIMURA, Masanari, et al. Palladium-Catalyzed, Triethylborane-Promoted C-allylation of Naphthols and Benzene Polyols by Direct Use of Allyl Alcohols. Synthesis. 2006, vol.21, p.3611-3616. and KUNTZ, Emile, et al. Palladium TPPTS catalyst in water: C-allylation of phenol and guaiacol with allyl alcohol and novel isomerisation of allyl ethers of phenol and guaiacol. Journal of Molecular Catalysis A: Chemical. 2006, vol.244, p.124-138. Kimura describes the direct C-allylation of nathols and benzene polyols, using a combination of a ctalystic amount of Pd(0) species and triethylborane as catalyst. Kuntz, on the other hand, produces the alkenylphenols through a fast transformation of ethers. lgnacia Fernández et al. discloses the C-allylation using dicationic Ru^{IV} Catalysts in FERNANDEZ, Ignacio, et al. High-Yield Ruthenium-Catalyzed Friedel-Crafts-Type Allylation Reactions Using Dicationic Ru(IV) Catalysts. Angew. Chem., Int. Ed. Engl.. 2006, vol.45, p.6386-6391. and FERNANDEZ, Ignacio, et al. Allylic Alcohols as Substrate for Ruthenium-Catalyzed C-C Coupling Allylation Reactions. Helvetica Chimica Acta. 2007, vol.90, p.271-276.

It should be noted that O-allylated products, if formed at all, are typically reversibly formed, while C-allylated products are produced irreversibly. C-allylation can thus be the predominant reaction because this yields the eventual thermodynamic end products, making it hard to produce allyl aryl ethers. The problem underlying the current invention is to improve the selective O-allylation in a manner that is environmentally friendly and highly atom-efficient, without generating saline waste. This problem has been solved, by use of transition metal complexes (in particular Ru and Pd) with a specific bidentate phosphine as ligand.

It should also be noted that bidentate phosphines and their synthesis have been disclosed in earlier patent references, such as EP 380162 on catalyst compositions suitable for the preparation of polymers of carbon monoxide and one or more olefinically unsaturated compounds. Likewise, tin CA 2086285 a process is found for the preparation of vicinally-disubstituted bis(diorganophosphino)alkanes, -alkenes, or -arenes, which are prepared by reacting a secondary phosphine with a vicinally disubstituted dihaloalkane, -alkene or -arene. Moreover, in WO 0187899 bidentate ligands are described having a bivalent aliphatic bridging group containing from 2 to 6 atoms in the bridge, which is substituted with two or more substituents. The catalyst system comprises: a) a source of group VIII metal cations, b) a source of such a bidentate ligand, and c) a source of anions. Also described is the use of such a catalyst system in a process for the carbonylation of optionally substituted ethylenically or acetylenically unsaturated compounds by reaction with carbon monoxide and a co-reactant. None of these references, however, discuss the preparation of O-allylated products.

### Disclosure of Invention

Accordingly, the invention provides a process for the preparation of an allyl aryl ether comprising the O-allylation of an aromatic hydroxyl containing compound with an allyl source in the presence of a catalyst, wherein the catalyst is a complex of a transition metal M with a bidentate diphosphine ligand of the formula X₂P-R-PX₂ wherein each X independently is a an aliphatic substituent having from 1 to 9 carbon atoms, a cycloaliphatic substituent having from 5 to 9 carbon atoms or an aromatic substituent having from 6 to 9 carbon atoms and R is a bridging group having 2 to 4 bridging atoms between the phosphorus atoms and wherein at least one of the bridging atoms is substituted (i.e., with an atom other than hydrogen). More specifically, the invention provides a process for the preparation of an allyl phenyl ether using a ruthenium-cyclopentadienyl complex (Ru-Cp, wherein Cp is a cyclopentadienyl group or a substituted cyclopentadienyl group) as catalyst.

Some of the metal complexes of the bidentate diphosphine ligands having a branched or substituted bridging group are novel. Accordingly these metal complexes have been claimed.

The allyl aryl ethers prepared by the process of the invention can be used, amongst others, as intermediate in the synthesis of epoxy resins. Hence the use of these ethers has also been claimed.

### Modes for Carrying Out the Invention

Where in this application reference is made to a catalyst, then such definition includes the precatalyst that is activated during the reaction, as well as the activating species in different oxidation states generated during the catalytic reaction. For instance, it is common to activate a Ru-Cp complex with silver (4-toluenesulfonate) to remove the strongly coordinating chloride anion present in the precatalyst. Instead of silver tosylate, also silver triflate (trifluoromethanesulfonate), silver tetrafluoroborate, silver hexafluorophosphate and similar non-coordinating silver salts may be used.

The aromatic hydroxyl containing compound is a phenolic compound having at least one aromatic nucleus and at least one hydroxyl group attached thereto. Unsubstituted phenol (C₆H₅OH) is the simplest species of this genus. 1,2- or 1,3- or 1,4-benzenediol and 1,3,5-benzene triol are the simplest species carrying more than one hydroxyl group. Moreover, the phenol may carry one or more inert substituents, such as e.g. *p*-cresol, 2,4,6-trimethylphenol or 4-*tert-*butylphenol. The aromatic nucleus may be part of a fused ring system, such as 1- or 2-naphthol, 1-, 2-, or 9-anthrol, which again may have more than 1 hydroxyl group (e.g., anthralin = 1,8,9-trihydroxyanthracene), or fused with a cycloaliphatic ring (s) such as for example indane, 1,2, 3,4-tetrahydronaphthalene and fluorene. Also suitable are phenolic compounds containing multinuclear aromatic rings, such as for example biphenyl, 2,2-diphenyl propane, bisphenylene oxide, tetrakis (1,1, 2,2-phenyl) ethane, stilbene, phenol-formaldehyde novolac, cresol-formaldehyde novolac, phenoldicyclopentadiene novolac and hyper-branched aromatic phenol dendrimers. The phenolic compound may also contain one or more heteroatoms such as for example O, N, S, Si, B or P; or any combination of these heteroatoms, such as for example, quinoxaline, thiophene and quinoline. Ar may also be a moiety containing mononuclear or multinuclear aromatic ring (s). Ar may also be a moiety containing mononuclear or multinuclear aromatic ring (s) fused with a cycloaliphatic ring (s) containing one or more heteroatoms such as for example O, N, S, Si, B or P; or any combination of these heteroatoms, such as for example, chroman, indoline and thioindane.

Of particular interest are phenolic compounds having 2 or more hydroxyl groups, which result in allyl aryl ethers that are multifunctional and hence suitable as intermediate in the synthesis of epoxy resins.

The process of the current invention aims at the production of allyl aryl ethers in an environmentally friendly and atom-efficient manner. As allyl source, allyl halides are preferably excluded, since these would result in the generation of a saline waste. To a lesser extent, also allyl acetates are preferably excluded. Rather, the preferred allyl source is either an allyl alcohol or an allyl ether. 2-Propen-1-ol is again the simplest species of an allyl alcohol. Other examples of suitable allyl alcohols include 3-buten-2-ol, cinnamyl alcohol (3-phenyl-2-propen-1-ol), and other alcohols of the general formula 1-R-2-propen-1-ol or 1-Ar-2-propen-1-ol or 1-RO-2-propen-1-ol or 1-ArO-2-propen-1-ol, or 3-R-2-propen-1-ol or 3-Ar-2-propen-1-ol or 3-RO-2-propen-1-ol or 3-ArO-2-propen-1-ol, wherein R represents an alkyl group, preferably of up to 6 carbon atoms, wherein O represents an oxygen atom, and wherein Ar represents an aryl group, preferably of up to 12 carbon atoms. These alcohols may be used by themselves, or in a mixture. Moreover, their diethers may be used, or ethers of a mixture of these alcohols. Moreover, a mixture of allyl alcohol(s) and allyl ether(s) may be used.

Preferably the allyl source is 2-propen-1-ol ("AA") or the ether thereof ("DAE"), or a combination of AA and DAE, most preferably AA.

The aromatic hydroxyl containing compound and the allyl source are preferably reacted in amounts sufficient to have at least one of the hydroxyl groups of the aromatic hydroxyl containing compound react with the allyl source. In case of a phenolic monool (e.g., phenol), and AA as the allyl source, the molar ratio between these reactants varies from 10:1 to 1:10, suitably from 5:1 to 1:5, preferably from 3:1 to 1:3, more preferably from 2:1 to 1:2. In case of DAE, the relative amount of aromatic hydroxyl containing compound is increased, as DAE may generate ideally two molecules of AA to react with the phenolic compound. In case of a phenolic polyol (e.g., bisphenol A or F) the relative amount of aromatic hydroxyl containing compound is decreased, corresponding to the higher hydroxyl content of said compound.

The catalyst systems of the current invention may also be used for the allylation of alkanols and/or aliphatic polyols.

The catalyst is a transition metal complex with a substituted or branched bidentate diphosphine ligand, and optionally comprising a coordinating species and/or a counterion, wherein M is a transition metal, L is a coordinating species, Y is a counterion. The bridging atoms are preferably selected from carbon atoms, but may also comprise a silicium atom. The bridging group present in the phosphorus bidentate ligands preferably contains three carbon atoms in the bridge.

Suitable transition metals include ruthenium, rhodium, rhenium, palladium, iridium, tungsten, molybdenum, chromium, platinum, nickel, tin, copper, osmium, iron, and preferably from ruthenium, osmium, and iron and more preferably is ruthenium. Various coordinating species L may be used, subject to the selection of the transition metal and the transition state of the metal. It is reiterated that the transition state of the metal as active catalyst may and frequently is different from that of the precatalyst. Coordinating species may include carbonyl groups, indenyl, cyclopentadienyl and optionally substituted cyclopentadienyl groups. Indeed, in case of a ruthenium complex, the coordinating species is preferably cyclopentadienyl or a substituted cyclopentadienyl group (e.g., Cp* = pentamethylcyclopentadienyl).

From VAN DER DRIFT et al., mentioned supra, bidentate ligands and Ru-Cp complexes based thereon are known. The diphosphines used as ligands all have unsubstituted aliphatic bridging groups between the phosphorus atoms. Some of the ligands used in this reference include bis(diphenylphosphino)methane (dppm); 1,2-bis(diphenylphosphino)ethane (dppe); 1,3-bis(diphenylphosphino)propane (dppp); 1 ,4-bis(diphenylphosphino)butane (dppb); 1,2-bis(diphenylphosphino)benzene (dppbz); *cis*-1,2-bis(diphenylphosphino)ethene (*cis*-dppv); 1,2-bis(di(*ortho*-methoxyphenylphosphino)ethane (*o-*MeO-dppe); and 1,2-bis(dicyclohexylphosphino)ethane (dcpe).

In view of its reactivity in the formation of DAE from AA, [RuClCp(*o*-MeO-dppe)] was chosen by VAN DER DRIFT for the reaction of AA with other (aromatic) nucleophiles. Surprisingly, it has been discovered that bidentate diphosphine ligands having 2 to 4 bridging atoms between the phosphorus atoms and wherein at least one of the bridging atoms is substituted with a group X clearly outperform the ligands disclosed by VAN DER DRIFT in terms of the combination of conversion and selectivity when used for O-allylation.

The bridge between the two phosphorus atoms is thus a key feature to achieve the improved combination of selectivity and conversion. The substituent on one or more of the bridging atoms of the bidentate diphosphine ligands may be: an alkyl or cycloalkyl group, with preferably up to 6 carbon atoms; an aryl group with preferably up to 9 carbon atoms; or a divalent alkenyl group, with preferably up to 6 carbon atoms, attached to the same bridging atom or another bridging atom. Preferably the substituent is a methyl, ethyl, phenyl or methylene group. The substituent may be substituted itself, e.g. with inert alkyl groups or functional groups and/or comprise one or more heteroatoms, such as O, P, N and S atoms. Preferably there are two substituents attached to the same or different bridging atom. In this case, the substituents are preferably methyl and/or ethyl groups.

The bridge may comprise two or three or four bridging atoms. Surprisingly good results in terms of selectivity have been achieved using a bidentate diphosphine ligand with three atoms in the bridge, e.g., a substituted 1,3-propylene group or a substituted dimethylenesilane group.

The substituents X attached to the phosphorus atoms may be independently selected from: alkyl groups, with preferably up to 6 carbon atoms; cycloalkyl or aryl groups with preferably up to 9 carbon atoms. Two substitutents X may also together form a divalent alkylene group, with from 3 up to 6 carbon atoms, attached to the same or different phosphorus atom(s). The cycloalkyl and/or aryl group may be substituted with one or more alkyl groups having up to 6 carbon atoms. The two phosphorus atoms may therefore be part of a 1,5-diphosphacyclooctane or similar substituted heterocycle. These substituents X may be also comprise one or more functional groups and/or have one or more heteroatoms as part of the group. Particularly suitable substituents X include phenyl, ortho-methoxyphenyl, ortho-ethoxylphenyl, and cyclohexyl.

Preferably the substituents X are identical.

Illustrative examples of suitable ligands therefore include.
- 1,3-bis(*ortho*-methoxyphenylphosphino)-2,2-dimethylpropane (o-MeOdppdmp)
- 1,3-bis(phenylphosphino)-2,2-dimethylpropane (dppdmp)
- 1,3-bis(phenylphosphino)-2,2-diethylpropane (dppdep)
- bis(diphenylphosphinomethyl)dimethylsilane (dppdms)
- 2,4-bis(phenylphosphino)-pentane (dpppt), and
- 1,3-bis(phenylphosphino)-isobutylene (dppib).

The catalyst is present during the reaction in a catalytically effective amount, preferably in an amount of from about 0.005 to about 200 mmoles of metal per mole of hydroxyl group(s) in the phenolic compound. More preferably, the catalyst is used in an amount of from about 0.05 to 20 mmoles per mole of hydroxyl group(s) in the phenolic compound. Indeed, for reasonable activity, most preferably small amounts of catalyst are used, such as about 0.1 mmole per mole of hydroxyl group(s) in the phenolic compound.

As mentioned before, the catalyst may need to be activated to perform the O-allylation. Ru-Cp complexes typically have a strongly coordinating chloride anion when obtained as precatalyst, which may be removed with silver tosylate, silver triflate or other non-coordinating anions such as silver tetrafluoroborate, silver hexafluorophosphate o create a vacant site for substrate coordination.

The catalyst may be immobilized, resulting in a heterogeneous catalyst. For instance, the catalyst (metal complex) can be immobilized on an ion-exchange resin with sulfonic acid groups. Examples of suitable resins include for example DOWEX™ WX4, Amberlyst™ 15, or Nafion™ NR 50.

The O-allylation products may be prepared in a continuous reaction or in a batch reaction.

In a preferred embodiment, the O-allylation reaction is performed in the presence of an acid, in particular in case of a bidentate diphosphine ligand having 2 or 3 bridging atoms. Thus, whereas VAN DER DRIFT et al, mentioned supra, found the selectivity towards C-allylation was improved, which would therefore be detrimental to the balance of activity and selectivity, the inventors surprisingly found a high activity and selectivity in the process of the invention when performed in the presence of an acid. Thus, the presence of 0.1 mol% to 25 mol%, preferably 0.15 to 20 mol%, more preferably 0.2 to 10 mol% and most preferably 1 to 5 mol% of acid on the amount of phenolic compound increases the O-allylation reaction rate whilst avoiding the preparation of C-allylated products. The acid may be selected on the basis of its logarithmic acid dissociation constant, pKa. The acid is preferably selected from the organic acids with a pKa < 5, preferably with a pKa < 3. Suitable acids include acetic acid and triflic acid and such, an in particular those acids whose silver salt have been used to activate the catalyst. The most preferred acid is p-toluenesulfonic acid.

The reaction is preferably carried out in an organic solvent. Although solvents like diethylene glycol dimethyl ether (diglyme) may be used, the solvent is more preferably an apolar solvent. The selection typically is done on the basis of the availability, cost and acceptability for environmental and health issues. The solvent has an effect on the total conversion rate of the reaction and the selectivity of the allylation (O-allylation versus C-allylation). The organic solvents may be selected from aromatic or aliphatic hydrocarbons, with the preferred solvent being toluene and the most preferred solvent being heptane.

The reactions are preferably conducted at a temperature between 60 and 150°C, preferably between 80 and 130°C and most preferably between 90 and 120°C. The following examples will more fully illustrate selected embodiments of this invention. All parts, percentages and proportions referred to herein and in the appended claims are by mol unless otherwise indicated.

### Examples

**Table 1, ligand abbreviations**

| Inventive | Comparative | Compound |
|---|---|---|
| o-MeOdppdmp | | 1,3-bis(*ortho*-methoxyphenylphosphino)-2,2-dimethylpropane |
| dppdmp | | 1,3-bis(phenylphosphino)-2,2-dimethylpropane |
| dppdep | | 1,3-bis(phenylphosphino)-2,2-diethylpropane |
| dppdms | | bis(diphenylphosphinomethyl)dimethylsilane |
| 2,4-dpppt | | 2,4-bis(phenylphosphino)-pentane |
| | | |
| dppib | | 1,3-bis(phenylphosphino)-isobutylene |
| dppbz | | 1,2-bis(diphenylphosphino)benzene, commercially available from Acros |
| 2,3-dppb | | 2,3-bis(phenylphosphino)-butane, commercially available from Strem Chemicals |
| | | |
| | dppe | 1,2-bis(diphenylphosphino)ethane |
| | dppp | 1,3-bis(diphenylphosphino)propane |
| | | |
| | | |
| | | |
| | o-MeO-dppe | 1,2-bis(di(*ortho*-methoxyphenylphosphino)ethane |
| | | |
| | | |

### Ligand synthesis

The diphosphine ligands dppbz and (S,S)-2,3-dppb were commercially available and used as received.

A modified protocol for the synthesis of the diphosphine ligands was used.¹

The ligands: dppdmp²; dppdep³; dppdms⁴; 2,4-dpppt³; dppib⁵; and *o*-MeOdppdmp⁶ were earlier described in literature. The ruthenium complexes [RuCpCl(PPh₃)₂]⁷; [RuCpCl(dppe)]⁸, [RuCpCl(dppp)]⁹ and [RuCpCl(dppbz)]¹⁰ were synthesized as reported.
¹ Hewertson, W.; Watson, H. R. *J Chem. Soc*. **1962**, 1490-&.
² Kraihanzel, C. S.; Ressner, J. M.; Gray, G. M. *Inorg. Chem*. **1982**, *21*, 879-887.
³ Bianchini, C.; Lee, H. M.; Meli, A.; Moneti, S.; Vizza, F.; Fontani, M.; Zanello, P. *Macromolecules* **1999**, *32*, 4183-4193.
⁴ de Groot, D.; Reek, J. N. H.; Kamer, P. C. J.; van Leeuwen, P. *Eur. J Org. Chem*. **2002**, 1085-1095.
⁵ Schmidbaur, H.; Paschalidis, C.; Steigelmann, O.; Muller, G. *Chem. Berichte* **1989**, *122*, 1851-1855.
⁶ Ginkel, R. v.; Made, A. v. d.; With, J. d.; Eilenberg, W. *Patent* **2003**, US 6548708B1*.*
⁷ Bruce, M. I.; Wong, F. S.; Skelton, B. W.; White, A. H. *J Chem. Soc.-Dalton Trans*. **1981**, 1398-1405.
⁸ Alonso, A. G.; Reventos, L. B. *J Organomet. Chem*. **1988**, *338*, 249-254.
⁹ van der Drift, R. C.; Gagliardo, M.; Kooijman, H.; Spek, A. L.; Bouwman, E.; Drent, E. *J. Organomet. Chem*. **2005,** *690*, 1044-1055.
¹⁰ Guan, H. R.; Iimura, M.; Magee, M. P.; Norton, J. R.; Janak, K. E., *Organometallics* **2003**, *22*, 4084

### General procedure for RuCpCl(PP) synthesis.

A solution of [RuCpCl(PPh₃)₂] (72 mg, 0.1 mmol) and the bidentate diphosphine ligand (0.1 mmol) in 5 ml toluene was stirred for 16 h at 90 °C. The solution was cooled to room temperature and flushed over a column of silica gel (3 g, d = 1 cm) with 15 ml of toluene to remove the triphenylphosphine. Finally, the orange product was eluted with ethyl acetate until the eluens was colorless. The solution was then concentrated in *vacuo* to approximately 1 ml and the product precipitated with petroleum ether.

RuCpCl(*o*-MeOdppdmp) was obtained as a yellow solid in a yield of 64 mg (84%). Anal. Calcd for C₃₈H₄₃ClO₄P₂Ru·1.5(toluene): C, 62.21; H, 5.91. Found: C, 62.20; H, 6.16. ¹H-NMR (CDCl₃): δ 7.37-7.12 (m, 8H, ArH), 7.03-6.93 (m, 7H, ArH), 6.69 (d, 4H, *J* = 8 Hz, ArH), 4.42 (s, 5H, Cp), 3.29 (s, 6H, OMe), 3.24 (s, 6H, OMe), 2.91-2.89 (m, 2H, CH₂), 2.62-2.60 (m, 2H, CH₂), 0.88 (s, 3H, Me), 0.25 (s, 3H, Me). ³¹P{1H}-NMR (acetone-d₆): δ 28.1 (s).

**RuCpCl(dppdmp)** was obtained as a yellow solid in a yield of 54 mg (84%). Anal. Calcd for C₃₄H₃₅ClP₂Ru·0.33(toluene): C, 64.86; H, 5.64. Found: C, 64.64; H, 6.13. ¹H-NMR (CDCl₃): δ 7.63-7.49 (m, 8H, ArH), 7.35-7.26 (m, 12H, ArH), 4.48 (s, 5H, Cp), 3.02-2.95 (m, 2H, PCH), 2.17-2.10 (m, 2H, PCH), 1.02 (s, 3H, Me), 0.18 (s, 3H, Me). ³¹P{¹H}-NMR (CDCl₃): δ 40.0 (s).

**RuCpCl(dppdep)** was obtained as a yellow solid in a yield of 53 mg (79%). Anal. Calcd for C₃₆H₃₉ClP₂Ru·0.25(hexane) .0.25(water): C, 65.05; H, 6.28. Found: C, 64.61; H, 6.34. ¹H-NMR (CDCl₃): δ 7.67-7.61 (m, 4H, ArH), 7.51-7.45 (m, 4H, ArH), 7.37-7.30 (m, 12H, ArH), 4.39 (s, 5H, Cp), 2.87-2.79 (m, 2H, CH₂P), 2.18-2.09 (m, 2H, CH₂P), 1.25 (q, 2H, *J* = 7 Hz, CH₂), 0.71 (q, 2H, *J* = 7 Hz, CH₂), 0.64 (t, 3H, *J* = 7 Hz, CH₃), 0.14 (t, 3H, *J* = 7 Hz, CH₃). ³¹P{¹H}-NMR (CDCl₃): δ 39.6 (s). **RuCpCl(dppdms)** was obtained as a yellow solid in a yield of 52 mg (81%). Anal.

Calcd for C₃₃H₃₅ClP₂Ru·1.25(water): C, 60.73; H, 5.79. Found: C, 60.35; H, 5.84. ¹H-NMR (CDCl₃): δ 7.70-7.64 (m, 4H, ArH), 7.37-7.25 (m, 16H, ArH), 4.21 (s, 5H, Cp), 2.33-2.26 (m, 2H, PCH), 1.54-1.44 (m, 2H, PCH), -0.03 (s, 3H, Me), -0.38 (s, 3H, Me). ³¹P{¹H}-NMR (CDCl₃): δ 42.0 (s).

**RuCpCl(dppib)** was obtained as a yellow solid in a yield of 40 mg (92%). Anal. Calcd for C₃₃H₃₁ClP₂Ru·0.33(hexane): C, 64.20; H, 5.49. Found: C, 63.78; H, 5.87. ¹H-NMR (CDCl₃): δ 7.76-7.71 (m, 4H, ArH), 7.36-7.35 (m, 8H, ArH), 7.26-7.10 (m, 8H, ArH), 4.68 (s, 2H, =CH₂), 4.42 (s, 5H, Cp), 3.68-3.59 (m, 2H, CH₂P), 3.22-3.12 (m, 2H, CH₂P). ³¹P{¹H}-NMR (CDCl₃): δ 43.0 (s).

**RuCpCl(2,4-dpppt)** was obtained as a yellow solid in a yield of 70 mg (90%). Anal. Calcd for C₃₄H₃₅ClP₂Ru·0.25(hexane): C, 64.25; H, 5.85. Found: C, 64.23; H, 6.14. ¹H-NMR (CDCl₃): δ 7.85-7.82 (m, 4H, ArH), 7.37-7.34 (m, 8H, ArH), 7.27-7.20 (m, 8H, ArH), 4.13 (s, 5H, Cp), 3.23-3.16 (m, 2H, PCH), 2.03-1.81 (m, 2H, CH₂), 1.13 (bs, 6H, CH₃). ³¹P{¹H}-NMR (CDCl₃): δ 51.8 (s).

### Reaction of 4-tert-butylphenol with allyl alcohol under catalysis of different complexes

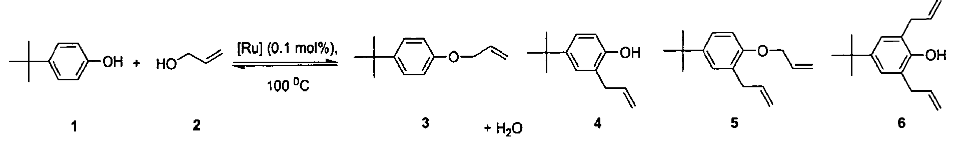

Allylation reactions were carried out in the manner shown above, at a molar ratio of 4-*tert*-butylphenol/allyl alcohol/catalyst/AgOTs = 1000/1000/1/2, using toluene as solvent, at 100 °C for 3 hours. The products were analyzed by GC and identified by GC-MS and NMR spectroscopy and the results are reported below.

### Comparative Example 1

**RuCpCl(*o*-MeOdppe)].** 2.5 mmol of 4-*tert*-butylphenol, 2.5 µmol of [RuCpCl(*o-*MeOdppe)] and 5 µmol of AgOTs were charged into the reaction vessel and flushed with argon. Degassed and dried toluene was added (3 ml) and the mixture was stirred for five minutes. Allyl alcohol was added (2.5 mmol) and the reaction was stirred for 3 hours at 100 °C. Conversion of **1**: 60%. Selectivity for: **3** 12%; **4** 74%; **5** 5%; **6** 9%.

### Comparative Example 2

**[RuCpCl(dppe)].** As example 1, but using [RuCpCl(dppe)] instead of [RuCpCl(*o-*MeOdppe)]. Conversion of **1**: 72%. Selectivity for: **3** 4%; **4** 56%; **5** 12%; **6** 28%.

### Comparative Example 3

[RuCpCl(dppp)]. As example 1, but using [RuCpCl(dppp)] instead of [RuCpCl(*o-*MeOdppe)]. Conversion of **1**: 53%. Selectivity for: **3** 44%; **4** 47%; **5** 6%; **6** 3%.

### Comparative Example 4

**[RuCpCl(dppe)].** As example 2, but 0.01 mmol of *p*-toluenesulfonic acid was added to the reaction mixture. Conversion of **1**: 67%. Selectivity for: **3** 0%; **4** 84%; **5** 0%; **6** 16%.

### Comparative Example 5

**[RuCpCl(dppp)].** As example 3, but 0.01 mmol of *p*-toluenesulfonic acid was added to the reaction mixture. Conversion of **1**: 80%. Selectivity for: **3** 2%; **4** 68%; **5** 4%; **6** 26%.

### Example 6

**[RuCpCl(dppbz)].** As example 2, but using [RuCpCl(dppbz)] instead of [RuCpCl(dppe)]. Conversion of **1**: 56%. Selectivity for: **3** 44%; **4** 56%.

### Example 7

**[RuCpCl(dppdmp)].** As example 3, but using [RuCpCl(dppdmp)] instead of [RuCpCl(dppp)]. Conversion of **1**: 30%. Selectivity for: **3** 87%; **4** 13%.

### Example 8

**[RuCpCl(dppdep)].** As example 3, but using [RuCpCl(dppdep)] instead of [RuCpCl(dppp)]. Conversion of **1**: 20%. Selectivity for: **3** 90%; **4** 10%.

### Example 9

**[RuCpCl(dppdms)].** As example 3, but using [RuCpCl(dppdms)] instead of [RuCpCl(dppp)]. Conversion of **1**: 20%. Selectivity for: **3** 99%; **4** 1%.

### Example 10

**[RuCpCl(*o*-MeOdppdmp)].** As example 3, but using [RuCpCl(*o*-MeOdppdmp)] instead of [RuCpCl(dppp)]. Conversion of **1**: 9%. Selectivity for: **3** 99%; **4** 1%.

### Example 11

**[RuCpCl(2,4-dpppt)].** As example 3, but using [RuCpCl(2,4-dpppt)] instead of [RuCpCl(dppp)]. Conversion of **1**: 25%. Selectivity for: **3** 86%; **4** 14%.

### Example 12

**[RuCpCl(dppdib)].** As example 3, but using [RuCpCl(2,4-dppdib)] instead of [RuCpCl(dppp)]. Conversion of **1**: 26%. Selectivity for: **3** 85%; **4** 15%.

### Example 13

**[RuCpCl(dppdmp)].** As example 5, but using [RuCpCl(dppdmp)] in stead of [RuCpCl(dppp)]. Conversion of **1**: 59%. Selectivity for: **3** 46%; **4** 39%; **5** 8%; **6** 7%.

### Example 14

**[RuCpCl(dppdep)].** As example 5, but using [RuCpCl(dppdep)] in stead of [RuCpCl(dppp)]. Conversion of **1**: 72%. Selectivity for: **3** 73%; **4** 20%; **5** 5%; **6** 2%.

### Example 15

**[RuCpCl(*o*-MeOdppdmp)].** As example 5, but using [RuCpCl(*o*-MeOdppdmp)] in stead of [RuCpCl(dppp)]. Conversion of **1**: 48%. Selectivity for: **3** 95%; **4** 5%.

### Example 16

**[RuCpCl(dppdmp)].** As example 7, but with diglyme as solvent instead of toluene. Conversion of **1**: 0%.

### Example 17

**[RuCpCl(dppdmp)].** As example 7, but with n-heptane as solvent instead of toluene. Conversion of **1**: 57%. Selectivity for: **3** 37%; **4** 46%; **5** 8%; **6** 9%.

### Example 18

**[RuCpCl(dppdmp)].** As example 17, but after one hour of stirring. Conversion of **1**: 54%. Selectivity for: **3** 84%; **4** 16%.

### Example 19

**[RuCpCl(dppdmp)].** As example 7, but with diallyl ether instead of allyl alcohol. Conversion of **1**: 9%. Selectivity for: 3 100%.

### Conclusions/observations

Comparative examples 1 to 3 use the catalyst system of VAN DER DRIFT et al. The product is mostly C-allylated. When acid is added, as in comparative examples 4 and 5, the selectivity for the O-allylated product (4) ion is even lessened.

Example 6 is the first example according to the present invention, having a substituted C2 bridge. The selectivity improved from only 4% to 44%.

Examples 7 to 12 all use a substituted C3 bridge. The selectivity for the O-allylated product improved from 44% to well more than 80%, with an exceptional high selectivity of 99% for dppdms as ligand (C3 bridge with a Silicon atom). Example 13 is similar to example 7, but performed in the presence of an acid, and similar to comparative example 5, but using a crossphos ligand. Whereas comparative example 5 showed the detrimental effect of acid on an unsubstituted ligand, as far as O-allylation is concerned, Example 13 versus example 7 shows a significant improvement in conversion with still a reasonable selectivity. Dppdep is the better ligand as compared to Dppdmp (cf. Examples 8 and 7). Example 14 illustrates that the conversion to the O-allylated product is significantly improved. The effect of the substituent on the phosphorus atoms is illustrated in Example 15. As compared to Example 13, the selectivity improved from 46% to 95% (despite a small reduction in the conversion.

Examples 16 and 17 illustrate the importance of the solvent. Diglyme, a polar solvent, is not a preferred solvent. Indeed the conversion was about 0%. On the other hand, using heptane as a solvent improved the conversion vis-à-vis the reaction in toluene (cf. example 7), albeit at a slight detriment in terms of the selectivity. These experiments do show, however, that with specific solvents or solvent blends, improved conversion and selectivity can be obtained.

Moreover, Example 18 illustrates that the selective still improves using n-heptane as a solvent to about the same levels as in Example 7 (but now with a conversion of 54% instead of 30%).

Example 19 is a scouting experiment with diallylether as allyl source. This experiment shows that DAE is not the inert product as VAN DER DRIFT et al found, but actually can be used in O-allylation reactions.

### Industrial Application

Intermediate for pharmaceuticals, e.g., wherein the O-allylation reaction is applied to provide a protecting group on a hydroxyl functionality in multistep synthesis of certain fine and/or pharmaceutical compounds.

### Intermediate for epoxy resins

### References cited in the description

*This list of references cited by the applicant is for the reader's convenience only. It does not form part of the European patent document. Even though great care has been taken in compiling the references, errors or omissions cannot be excluded and the EPO disclaims all liability in this regard.*
**Patent documents cited in the description**
   - US 4740330 (Page 1, lines 14, 32 and 33)
   - US 7049388 (Page 1, line 23)
   - EP 380162 (Page 4, line 5)
   - CA 2086285 (Page 4, line 7)
   - WO 0187899 (Page 4, line 11)
   - US 6548708 B1 (Page 12, line 2 and footnote 6)
**Non-patent documents cited in the description**
   - VAN DER DRIFT, Robert C., et al. Two reactions of allylic alcohols catalysed by ruthenium cyclopentadienyl conplexes with didentate phosphine ligands: isomerisation and ether formation. Journal of Molecular Catalysis A: Chemical. 2000, vol.159, p.163-177. (Page 2, line 1, page 7, line 22, page 9, line 28)
   - SABURI, Hajime, et al. Catalytic Dehydrative Allylation of Alcohols. Angew. Chem., Int. Ed. Engl.. 2005, vol.44, p.1730-1732. (Page 2, line 16)
   - SATOH, Tetsuya, et al. Palladium-Catalyzed Etherification of Allyl Alcohols Using Phenols int he Presence of Titanium(IV) Isopropoxide. J. org. chem.. 1997, vol.62, p.4877-4879. (Page 2, line 26)
   - KIM, Hahn, et al. A Mild and Efficient Method for the Stereoselective Formation of C-O Bonds: Palladium-Catalyzed Allylic Etherification Using Zinc(II) Alkoxides. Organic Letters. 2002, vol.4, no.24, p.4369-4371. (Page 2, line 32)
   - BRUNEAU, Christian, et al. Pentamethylcyclopentadienyl-Ruthenium Catalysts for Regio- and enantioselective allylation of Nucleophiles. Chem. Eur. J.. 2006, vol.12, p.5178-5187. (Page 3, line 7)
   - KIMURA, Masanari, et al. Palladium-Catalyzed, Triethylborane-Promoted C-allylation of Naphthols and Benzene Polyols by Direct Use of Allyl Alcohols. Synthesis. 2006, vol.21, p.3611-3616. (Page 3, line 14)
   - KUNTZ, Emile, et al. Palladium TPPTS catalyst in water: C-allylation of phenol and guaiacol with allyl alcohol and novel isomerisation of allyl ethers of phenol and guaiacol. Journal of Molecular Catalysis A: Chemical. 2006, vol.244, p.124-138. (Page 3, line 16)
   - FERNANDEZ, Ignacio, et al. High-Yield Ruthenium-Catalyzed Friedel-Crafts-Type Allylation Reactions Using Dicationic Ru(IV) Catalysts. Angew. Chem., Int. Ed. Engl.. 2006, vol.45, p.6386-6391. (Page 3, line 24)
   - FERNANDEZ, Ignacio, et al. Allylic Alcohols as Substrate for Ruthenium-Catalyzed C-C Coupling Allylation Reactions. Helvetica Chimica Acta. 2007, vol.90, p.271-276. (Page 3, line 26)
   - Hewertson, W.; Watson, H. R. J. Chem. Soc. 1962, 1490-&. (Page 12, line 1)
   - Kraihanzel, C. S.; Ressner, J. M.; Gray, G. M. Inorg. Chem. 1982, 21, 879-887. (Page 12, line 2)
   - Bianchini, C.; Lee, H. M.; Meli, A.; Moneti, S.; Vizza, F.; Fontani, M.; Zanello, P. Macromolecules 1999, 32, 4183-4193. (Page 12, line 2)
   - de Groot, D.; Reek, J. N. H.; Kamer, P. C. J.; van Leeuwen, P. Eur. J. Org. Chem. 2002, 1085-1095. (Page 12, line 2)
   - Schmidbaur, H.; Paschalidis, C.; Steigelmann, O.; Muller, G. Chem. Berichte 1989, 122, 1851-1855. (Page 12, line 2)
   - Bruce, M. I.; Wong, F. S.; Skelton, B. W.; White, A. H. J. Chem. Soc.-Dalton Trans. 1981, 1398-1405. (Page 12, line 3)
   - Alonso, A. G.; Reventos, L. B. J. Organomet. Chem. 1988, 338, 249-254. (Page 12, line 4)
   - van der Drift, R. C.; Gagliardo, M.; Kooijman, H.; Spek, A. L.; Bouwman, E.; Drent, E. J. Organomet. Chem. 2005, 690, 1044-1055. (Page 12, line 4)
   - Guan, H. R.; limura, M.; Magee, M. P.; Norton, J. R.; Janak, K. E., Organometallics 2003, 22, 4084 (Page 12, line 4)

## Claims

1. A process for the preparation of an allyl aryl ether comprising the O-allylation of an aromatic hydroxyl containing compound with an allyl source in the presence of a suitable catalyst, wherein the catalyst is a complex of the transition metal M with a bidentate diphosphine ligand of the formula X₂P-R-PX₂ wherein each X independently is a an aliphatic substituent having from 1 to 9 carbon atoms, a cycloaliphatic substituent having from 5 to 9 carbon atoms or an aromatic substituent having from 6 to 9 carbon atoms , or wherein two substituents X together form a divalent alkylene group, with from 3 up to 6 carbon atoms, attached to the same or different phosphorus atom(s), and R is a bridging group having 2 to 4 bridging atoms between the phosphorus atoms and wherein at least one of the bridging atoms is substituted.

2. The process of claim 1 wherein M is selected from the transition metals ruthenium, rhodium, rhenium, palladium, iridium, tungsten, molybdenum, chromium, platinum, nickel, tin, copper, osmium, iron, and preferably from ruthenium, osmium, and iron and more preferably is ruthenium.

3. The process of claim 1 or 2, wherein a ruthenium-cyclopentadienyl complex (Ru-Cp, wherein Cp is a cyclopentadienyl group or a substituted cyclopentadienyl group) is used as catalyst.

4. The process of any one of claims 1 to 3, wherein the ligand has three bridging atoms, preferably 3 carbon atoms or 2 carbon atoms and a silicium atom.

5. The process of any one of claims 1 to 4, wherein one or more of the bridging atoms within the ligand is or are substituted with a substituent selected from: an alkyl or cycloalkyl group, with preferably up to 6 carbon atoms; an aryl group with preferably up to 9 carbon atoms; or a divalent alkenyl group, with preferably up to 6 carbon atoms, attached to the same bridging atom or an other bridging atom, which groups may comprise one or more alkyl groups or functional groups substituted on said groups and/or comprise one or more heteroatoms selected from O, P, N and S atoms.

6. The process of any one of claims 1 to 5, wherein one or more of the bridging atoms within the ligand is or are substituted with a substituent selected from: methyl, ethyl, phenyl or methylene group, preferably with two methyl groups or two ethyl groups attached to a bridging atom or atoms.

7. The process of any one of claims 1 to 6, wherein each X independently is selected from: alkyl groups with up to 6 carbon atoms; aryl groups with up to 9 carbon atoms; or divalent alkenyl groups, with from 3 up to 6 carbon atoms, attached to the same or different phosphorus atom, preferably phenyl, ortho-methoxyphenyl, ortho-ethoxylphenyl, and cyclohexyl.

8. The process of any one of claims 1 to 7, wherein the ligand is one of: 1,3-bis(*ortho*-methoxyphenylphosphino)-2,2-dimethylpropane (o-MeOdppdmp) 1 ,3-bis(phenylphosphino)-2,2-dimethylpropane (dppdmp); 1,3-bis(phenylphosphino)-2,2-diethylpropane (dppdep); bis(diphenylphosphinomethyl)dimethylsilane (dppdms); 2,4-bis(phenylphosphino)-pentane (dpppt), and 1,3-bis(phenylphosphino)-isobutylene (dppib).

9. The process of any one of claims 1 to 8, wherein the catalyst is present during the reaction in a catalytically effective amount, preferably in an amount of from about 0.005 to about 200 mmoles, more preferably of from about 0.05 to about 20 mmoles more preferably about 0.1 mmole of metal per mole of hydroxyl group(s) in the aromatic hydroxyl containing compound.

10. The process of any one of claims 1 to 9, wherein the aromatic hydroxyl containing compound is a phenolic compound having at least one aromatic nucleus and at least one hydroxyl group attached thereto, or a mixture of such compounds, which compound or mixture of compounds comprises substituted or unsubstituted phenols, aromatic hydroxyl containing compounds having a fused ring system, aromatic hydroxyl containing compounds containing multiple aromatic rings, and aromatic hydroxyl containing compounds contain one or more heteroatoms selected from O, N, S, Si, B or P; or any combination of these heteroatoms.

11. The process of any one of claims 1 to 10, wherein the allyl source is an allyl alcohol or mixture of allyl alcohols of the general formulae
1-R-2-propen-1-ol or
1-Ar-2-propen-1-ol or
1-RO-2-propen-1-ol or
1-ArO-2-propen-1-ol, or
3-R-2-propen-1-ol or
3-Ar-2-propen-1-ol or
3-RO-2-propen-1-ol or
3-ArO-2-propen-1-ol,
wherein R represents an alkyl group, preferably of up to 6 carbon atoms,
wherein O represents an oxygen atom, and wherein Ar represents an aryl group, preferably of up to 12 carbon atoms and/or an ether of the allyl alcohol or allyl alcohols, preferably 2-propen-1-ol ("AA") or the ether thereof ("DAE") or the mixture of AA and DAE, preferably AA.

12. The process of any one of claims 1 to 11, wherein the allyl alcohol and the aromatic hydroxyl containing compound are used in a molar ratio of allyl alcohol versus the aromatic hydroxyl containing compound of from 1:10 to 10:1, preferably of from 1:5 to 5:1, more preferably of from 1:2 to 2:1, most preferably about 1:1.

13. The process of any one of claims 1 to 12, carried out in the presence of an acid, wherein the bidentate diphosphine ligand has preferably 2 or 3 bridging atoms between the phosphorus atoms.

14. The process of claim 13, wherein an acid with pKa < 5, preferably a pKa < 3, preferably selected from acetic acid, triflic acid and p-toluenesulfonic acid, more preferably p-toluenesulfonic acid is present in an amount of from 0.1 mol% to 25 mol%, preferably 0.15 to 20 mol%, more preferably 0.2 to 10 mol% and most preferably 1 to 5 mol% of acid calculated on the amount of the aromatic hydroxyl containing compound.

15. A transition metal complex with a bidentate diphosphine ligand, selected from
RuCpCl(*o*-MeOdppdmp); RuCpCl(dppdmp); RuCpCl(dppdep);
RuCpCl(dppdms); RuCpCl(dppib) and RuCpCl(2,4-dpppt).

16. Process for the preparation of epoxy resins wherein as intermediate use is made of the allyl aryl ethers prepared by the process of any one of claims 1 to 14.
